# EUROPÄISCHE PATENTSCHRIFT

(11) **EP 1 809 652 B1**
(45) Veröffentlichungstag und Bekanntmachung des Hinweises auf die Patenterteilung: **05.05.2010**
(21) Anmeldenummer: 05796353.0
(22) Anmeldetag: 01.11.2005
(51) Int. Cl.: C07K 5/08, C07K 5/06, A61K 38/06, A61K 38/05

(54) **NEUE TOPISCH ANWENDBARE WIRKSTOFFE GEGEN MIMISCHE UND ALTERABEDINGT FALTEN**
NOVEL TOPICAL APPLICATION AGENTS AGAINST MIMIC AND AGE-RELATED WRINKLES
NOUVEAUX PRINCIPES ACTIFS POUR APPLICATION TOPIQUE CONTRE LES RIDES D'EXPRESSION ET CELLES LIEES AU VIEILLISSEMENT

(30) Priorität: 02.11.2004 CH 18092004
(43) Veröffentlichungstag der Anmeldung: 25.07.2007
(73) Patentinhaber: DSM IP Assets B.V., 6411 TE Heerlen (NL)
(72) Erfinder: IMFELD, Dominik, CH-4142 Münchenstein (CH); ZIEGLER, Hugo, CH-4108 Witterswil (CH); WIKSTROEM, Peter, CH-5073 Gipf-Oberfrick (CH)
(74) Vertreter: Braun, André jr.
(86) Internationale Anmeldenummer: PCT/CH2005/000638
(87) Internationale Veröffentlichungsnummer: WO 2006/047900

(56) Entgegenhaltungen:
- EP-A- 1 180 524
- US-B1- 6 169 074

## Beschreibung

Falten entstehen meistens dadurch, dass sich die Muskeln sehr stark zusammenziehen bzw. in der stark zusammengezogenen Position sehr lange verharren.

Zur Behandlung von solchen mimischen und altersbedingten Falten wird heute oft Botox (Botulinumtoxin A) eingesetzt. Botox wird in die Muskeln injiziert und lähmt dadurch die Muskeln. Die Muskeln an den Augen oder an der Stirn werden dadurch ausser Gefecht gesetzt; ein Stirnrunzeln ist danach nicht mehr möglich. Dass die Behandlung mit Botox, welches unter die Haut gespritzt wird, von einem Arzt vorgenommen werden muss, der damit verbundene hohe Preis und dessen extrem hohe Toxizität sind jedoch schwer wiegende Nachteile von Botox. Die Wirkung hält 3 bis 6 Monate an, danach muss die Behandlung wiederholt werden.

Die Wirkungsweise von Botox besteht im selektiven Blockieren der Acetylcholin-Freisetzung an der neuromuskulären Synapse, wodurch eine Lähmung des Muskels eintritt. Dies wird erreicht durch Spaltung eines Proteins, genannt SNAP-25.

Die N-terminale Aminosäuresequenz von SNAP-25 (H-Glu-Glu-Met-Gln-Arg-Arg-NH₂) hemmt ebenfalls die Ca⁺⁺-abhängige Neurotransmitterfreisetzung in den Synapsen und führt zu einer Muskelrelaxation (EP1 180 524). Die daraus entwickelte, topisch applizierbare Verbindung Ac-Glu-Glu-Met-Gln-Arg-Arg-NH₂ ("Produkt A") wirkt 5000 mal schwächer als Botox, ist deshalb besser dosierbar und kaum toxisch. Allerdings ist seine Muskel relaxierende Wirkung zu schwach und zu unkonstant, um eine befriedigende Falten reduzierende Wirkung zu gestatten. Ein weiterer Nachteil ist dessen ungenügende proteolytische Stabilität.

Neben der durch Botox inhibierten Freisetzung des Neurotransmitters Acetylcholin gibt es zwei weitere Arten von Inhibitoren der Acetylcholin-Aktivität an der neuromuskulären Synapse: Inhibitoren des Acetylcholin-Rezeptors (z.B. Antihistamine, Atropin) und der Acetylcholinesterase (z.B. insektizide Phosphorsäureester).

Ziel der vorliegenden Erfindung war, als topische Wirkstoffe gegen mimische und altersbedingte Falten einsetzbare Muskel relaxierende Verbindungen zu finden, deren Wirkung auf der Hemmung des Acetylcholin Rezeptors beruht und welche nicht mit den Nachteilen von Botox und des Produkts A behaftet sind.

Überraschenderweise ist es nun gelungen, neue Tripeptide, tripeptidartige Verbindungen und Derivate davon (im weiteren "erfindungsgemässe Verbindungen" genannt) und topisch applizierbare Zusammensetzungen zur Behandlung von mimischen und altersbedingten Falten bereitzustellen, welche schnell und in ausreichender Konzentration zu ihrem Wirkort, der neuromuskulären Synapse, gelangen, diese blockieren und dadurch eine Muskel relaxierende Wirkung induzieren.

Dabei zeigt sich, dass die erfindungsgemässen Verbindungen ein deutlich besseres Wirkungsprofil hinsichtlich Muskel relaxierender Aktivität und eine erhöhte proteolytische Stabilität gegenüber der Vergleichsverbindung Produkt A aufweisen. Sie lassen sich auf Grund ihres relativ niederen Molekulargewichts und geeigneten Wasse.r/n-Oktanol-Verteilungskoeffizienten logP topisch applizieren.

Die vorliegende Erfindung betrifft Tripeptide, tripeptidartige Verbindungen und Derivate davon der allgemeinen Formel (I) 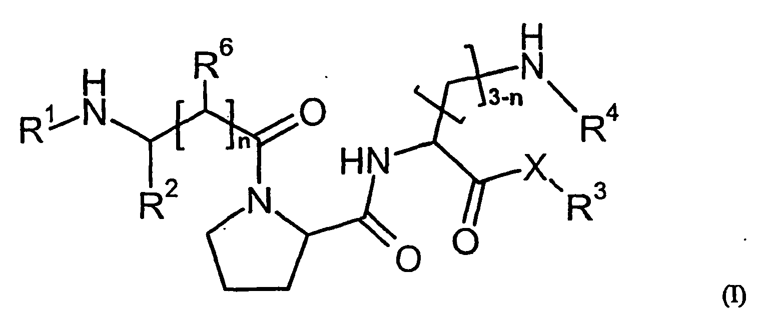 worin
X eine Bindung oder NH-CH(C=O)-(CH₂)₃₊ₙ-NH-R⁵,
n 0, 1 oder 2,
R¹, R⁴ und R⁵ unabhängig voneinander Wasserstoff, gegebenenfalls substituiertes C₁-C₆-Alkyl, Amidino oder Tetra-C₁-C₆-alkylamidinium,
R² Wasserstoff oder gegebenenfalls substituiertes C₁-C₆-Alkyl,
oder
R¹ und R² zusammen mit dem Rest, an den sie gebunden sind, einen 5- bis 7-gliedrigen, gesättigten Ring,
R³ C₁-C₁₂-Alkoxy, C₁-C₁₂-Alkylamino, gegebenenfalls substituiertes Aryl-C₁-C₆-alkylamino, gegebenenfalls substituiertes Heteroaryl-C₁-C₆-alkylamino, gegebenenfalls substituiertes Aryl-C₁-C₆-alkoxy oder gegebenenfalls substituiertes Heteroaryl-C₁-C₆-alkoxy und
R⁶ Wasserstoff oder, wenn n 1 ist, auch Amino oder zusammen mit R¹ und dem Rest, an den R⁶ und R¹ gebunden sind, einen 5- bis 7-gliedrigen, gesättigten Ring bedeuten,
als Racemate oder in ihrer enantiomerenreinen Form, sowie deren Salze.

Die vorliegende Erfindung betrifft weiterhin die Verwendung der vorstehend definierten Verbindungen und Salze als topisch anwendbare Wirkstoffe bzw. zur Herstellung topisch anwendbarer Zubereitungen, sowie topisch anwendbare Zubereitungen, enthaltend mindestens eine der vorstehend definierten Verbindungen oder ein Salz davon.

Die oben verwendeten allgemeinen Ausdrücke sind wie folgt definiert: Unter "Alkyl" als Gruppe per se und als Strukturelement für Alkyl enthaltende Gruppen sind sowohl lineare wie verzweigte gesättigte Kohlenwasserstoffreste zu verstehen. Beispiele sind Methyl, Ethyl, Propyl, n-Butyl, n-Pentyl, n-Hexyl, n-Heptyl, n-Octyl, n-Nonyl, n-Undecanyl, n-Dodecanyl, n-Tridecanyl, n-Hexadecanyl, n-Heptadecanyl, n-Octadecanyl und n-Nonadecanyl als unverzweigte und Isopropyl, tert.Butyl, Isobutyl, sec.Butyl und Isoamyl als verzweigte.

"Aryl" steht für aromatische Kohlenwasserstoffreste, wie Phenyl und Naphthyl; bevorzugt ist Phenyl.

"Heteroaryl" bezeichnet 5- bis 11-gliedrigen aromatische Ringsysteme, bestehend aus einem oder zwei Ringen, in denen 1 bis 3 Glieder Heteroatome sind, ausgewählt aus Sauerstoff, Schwefel und Stickstoff; 1 bis 2 Benzolringe können an den Heterocyclus ankondensiert sein. Beispiele sind Pyidyl, Pyrimidinyl, Pyridazinyl,: Pyrazinyl, 1,3,5-Triazinyl, Chinolinyl, Isochinolinyl Chinoxalinyl, Chinazolinyl, Phthalazinyl, Pyrrolyl, Pyrazolinyl, Imidazolinyl, 1,2,4-Triazolinyl, Tetrazolinyl, Furyl, Thienyl, Oxazolinyl, Thiazolinyl, Isothiazolinyl, Benzoxazolyl, Benzothienyl, Indolyl, Benzimidazolyl. Indazolyl, Benzotriazolyl und Benzothiazolyl, wobei die Verknüpfung entweder am Heteroteil oder am Benzoteil und bei den π-Überschussheteroaromaten über den Stickstoff oder über einen beliebigen Kohlenstoff erfolgen kann.

Beispiele für den 5- bis 7-gliedrigen, gesättigten Ring, den R¹ und R² bzw. R¹ und R⁶ zusammen mit dem Rest, an den sie gebunden sind, bilden können, sind Pyrrolidinyl, Piperidinyl, Piperazinyl, Morpholinyl, Azepinyl, Oxazolidinyl, Thiazolidinyl und 1,2,3,4-Tetrahydrochinolinyl.

Substituenten der gegebenenfalls substituierten Alkylreste und der diese Reste enthaltenden Gruppen sind beispielsweise Halogen, Amino, Guanidino, Hydroxy, C₁-C₆-Alkoxy, C₁-C₆-Alkylthio, Carboxy, Carbamoyl, gegebenenfalls substituiertes Phenyl, gegebenenfalls substituiertes Benzyl, Imidazolylmethyl, Indolylmethyl und Cyano.

Substituenten der gegebenenfalls substituierten Aryl- und Heteroarylgruppen sind z.B. Halogen, C₁-C₆-Alkyl, Hydroxy, C₁-C₆-Alkoxy, C₁-C₆-Alkoxycarbonyl , CN, Amino, C₁-C₆-Alkylamino, Di-C₁-C₆-alkylamino, Aminocarbonyl, C₁-C₆-Alkylaminocarbonyl, Di-C₁-C₆-alkylaminocarbonyl, C₁-C₆-Alkylthio, C₁-C₆-Alkylsulfoxyl, C₁-C₆-Alkylsulfonyl, gegebenenfalls substituiertes Benzyl, gegebenenfalls substituiertes Phenyl, gegebenenfalls substituiertes Phenoxy oder gegebenenfalls substituiertes Phenylcarbonyl, wobei die oben erwähnten aromatischen Ringe substituiert sein können mit 1 bis 3 identischen oder unterschiedlichen Substituenten, ausgewählt aus der Gruppe bestehend aus Halogen, Cyano, C₁-C₆-Alkyl, C₁-C₆-Alkoxy, Hydroxy, Amino, C₁-C₆-Alkylamino, Di-C₁-C₆-alkylamino und C₁-C₆-Alkoxycarbonyl.

"Halogen" steht für Fluor, Chlor, Brom und Jod; bevorzugt sind Fluor und Chlor.

In Formel 1 bedeuten R¹ vorzugsweise Wasserstoff, R² bedeutet vorzugsweise Wasserstoff oder Methyl, R³ bedeutet vorzugsweise Aryl-C₁-C₆-alkylamino und n bedeutet vorzugsweise 0 oder 1. Besonders bevorzugt sind Verbindungen der Formel I, worin R¹ Wasserstoff, R² Wasserstoff oder Methyl, R³ Aryl-C₁-C₆-alkylamino und n 0 oder 1 bedeuten.

Besonders bevorzugte erfindungsgemässe Verbindungen sind
- H-Ala-Pro-Arg-Arg-NH-benzyl;
- H-(β-Ala)-Pro-Dab-NH-benzyl;
- H-Dap-Pro-Arg-NH-benzyl;
- H-Ala-Pro-Arg-NH-(CH₂)₂-phenyl;
- H-(β-Ala)-Pro-Gab-NH-benzyl;
- N-[bis(dimethylamino)methylen]-Ala-Pro-Arg-NH-benzyl; und
   Säureadditionssalze dieser Verbindungen.

Die Einzelverbindungen in den folgenden Tabellen 1 bis 3 illustrieren die Erfindung.

**Tabelle 1: Verbindungen der Formel (I), worin X eine Bindung bedeutet**

| **Nr** | **R¹** | **R²** | **R³** | **R⁴** | **n** | **R⁶** |
|---|---|---|---|---|---|---|
| 1 | H | H | CH₃ | H | 0 | - |
| 2 | H | H | OBenzyl | H | 0 | - |
| 3 | H | H | O(CH₂)₂Phenyl | H | 0 | - |
| 4 | H | H | O-3,5-F₂-Benzyl | H | 0 | - |
| 5 | H | H | OCH₂-2-Pyridyl | H | 0 | - |
| 6 | H | H | NH(CH₂)₅CH₃ | H | 0 | - |
| 7 | H | H | NHBenzyl | H | 0 | - |
| 8 | H | H | NH(CH₂)₂Phenyl | H | 0 | - |
| 9 | H | H | NH-3,5-F₂-Benzyl | H | 0 | - |
| 10 | H | H | NHCH₂-3-Pyridyl | H | 0 | - |
| 11 | H | CH₃ | OCH₃ | H | 0 | - |
| 12 | H | CH₃ | OBenzyl | H | 0 | - |
| 13 | H | CH₃ | O(CH₂)₂Phenyl | H | 0 | - |
| 14 | H | CH₃ | 0-3,5-F₂-Benzyl | H | 0 | - |
| 15 | H | CH₃ | OCH₂-2-Pyridyl | H | 0 | - |
| 16 | H | CH₃ | NH(CH₂)₅CH₃ | H | 0 | - |
| 17 | H | CH₃ | NHBenzyl | H | 0 | - |
| 18 | H | CH₃ | NH(CH₂)₂Phenyl | H | 0 | - |
| 19 | H | CH₃ | NH-4-Cl-Benzyl | H | 0 | - |
| 20 | H | CH₃ | NHCH₂-3-Pyridyl | H | 0 | - |
| 21 | H | CH₂OH | OCH₃ | H | 0 | - |
| 22 | H | CH₂OH | OBenzyl | H | 0 | - |
| 23 | H | CH₂OH | O(CH₂)₂Phenyl | H | 0 | - |
| 24 | H | CH₂OH | O-3,5-F₂-Benzyl | H | 0 | - |
| 25 | H | CH₂OH | OCH₂-2-Pyridyl | H | 0 | - |
| 26 | H | CH₂OH | NH(CH₂)₅CH₃ | H | 0 | - |
| 27 | H | CH₂OH | NHBenzyl | H | 0 | - |
| 28 | H | CH₂OH | NH(CH₂)₂Phenyl | H | 0 | - |
| 29 | H | CH₂OH | NH-4-Cl-Benzyl | H | 0 | - |
| 30 | H | CH₂OH | NHCH₂-3-Pyridyl | H | 0 | - |
| 31 | H | (CH₂)₂NH₂ | OCH₃ | H | 0 | - |
| 32 | H | (CH₂)₂NH₂ | OBenzyl | H | 0 | - |
| 33 | H | (CH₂)₂NH₂ | O(CH₂)₂Phenyl | H | 0 | - |
| 34 | H | (CH₂)₂NH₂ | O-3,5-F₂-Benzyl | H | 0 | - |
| 35 | H | (CH₂)₂NH₂ | OCH₂-2-Pyridyl | H | 0 | - |
| 36 | H | (CH₂)₂NH₂ | NH(CH₂)₅CH₃ | H | 0 | - |
| 37 | H | (CH₂)₂NH₂ | NHBenzyl | H | 0 | - |
| 38 | H | (CH₂)₂NH₂ | NH(CH₂)₂Phenyl | H | 0 | - |
| 39 | H | (CH₂)₂NH₂ | NH-4-Cl-Benzyl | H | 0 | - |
| 40 | H | (CH₂)₂NH₂ | NHCH₂-3-Pyridyl | H | 0 | - |
| 41 | H | (CH₂)₃NH₂ | OCH₃ | H | 0 | - |
| 42 | H | (CH₂)₃NH₂ | OBenzyl | H. | 0 | - |
| 43 | H | (CH₂)₃NH₂ | O(CH₂)₂Phenyl | H | 0 | - |
| 44 | H | (CH₂)₃NH₂ | O-3,5-F₂-Benzyl | H | 0 | - |
| 45 | H | (CH₂)₃NH₂ | OCH₂-2-Pyridyl | H | 0 | - |
| 46 | H | (CH₂)₃NH₂ | NH(CH₂)₅CH₃ | H | 0 | - |
| 47 | H | (CH₂)₃NH₂ | NHBenzyl | H | 0 | - |
| 48 | H | (CH₂)₃NH₂ | NH(CH₂)₂Phenyl | H | 0 | |
| 49 | H | (CH₂)₃NH₂ | NH-4-Cl-Benzyl | H | 0 | - |
| 50 | H | (CH₂)₃NH₂ | NHCH₂-3-Pyridyl | H | 0 | - |
| 51 | H | CH₂NH₂ | NHBenzyl | H | 0 | - |
| 52 | H | (CH₂)₄NH₂ | NHBenzyl | H | 0 | - |
| 53 | H | (CH₂)₃NHC=NH(N H₂) | NHBenzyl | H | 0 | - |
| 54 | H | CH₂CH₃ | NHBenzyl | H | 0 | - |
| 55 | H | (CH₂)₂CH₃ | NHBenzyl | H | 0 | - |
| 56 | H | CH(CH₃)₂ | NHBenzyl | H | 0 | - |
| 57 | H | CH₂CH(CH₃)₂ | NHBenzyl | H | 0 | - |
| 58 | H | CH(CH₃)CH₂CH₃ | NHBenzyl | H | 0 | - |
| 59 | H | (CH₂)₂SCH₃ | NHBenzyl | H | 0 | - |
| 60 | H | CH₂COOH | NHBenzyl | H | 0 | - |
| 61 | H | CH₂CONH₂ | NHBenzyl | H | 0 | - |
| 62 | H | (CH₂)₂OH | NHBenzyl | H | 0 | - |
| 63 | H | CH(CH₃)OH | NHBenzyl | H | 0 | - |
| 64 | H | (CH₂)₂COOH | NHBenzyl | H | 0 | - |
| 65 | H | (CH₂)₂CONH₂ | NHBenzyl | H | 0 | - |
| 66 | H | CH₂Phenyl | NHBenzyl | H | 0 | - |
| 67 | H | CH₂-4-OH-Phenyl | NHBenzyl | H | 0 | - |
| 68 | H | Phenyl | NHBenzyl | H | 0 | - |
| 69 | H | CH₂-4-Imidazolyl | NHBenzyl | H | 0 | - |
| 70 | H | CH₂-3-Indolyl | NHBenzyl | H | 0 | - |
| 71 | H | H | NHBenzyl | Benzyl | 0 | - |
| 72 | H | CH₃ | NHBenzyl | Benzyl | 0 | - |
| 73 | H | CH₂OH | NHBenzyl | Benzyl | 0 | - |
| 74 | H | (CH₂)₂NH₂ | NHBenzyl | Benzyl | 0 | - |
| 75 | H | (CH₂)₃NH₂ | NHBenzyl | Benzyl | 0 | - |
| 76 | H | CH₂NH₂ | NHBenzyl | Benzyl | 0 | - |
| 77 | H | (CH₂)₄NH₂ | NHBenzyl | Benzyl | 0 | - |
| 78 | H | (CH₂)₃NHC=NH(N H₂) | NHBenzyl | Benzyl | 0 | - |
| 79 | H | CH₂CH₃ | NHBenzyl | Benzyl | 0 | - |
| 80 | H | (CH₂)₂CH₃ | NHBenzyl | Benzyl | 0 | - |
| 81 | H | CH(CH₃)₂ | NHBenzyl | Benzyl | 0 | - |
| 82 | H | CH₂CH(CH₃)₂ | NHBenzyl | Benzyl | 0 | - |
| 83 | H | CH(CH₃)CH₂CH₃ | NHBenzyl | Benzyl | 0 | - |
| 84 | H | (CH₂)₂SCH₃ | NHBenzyl | Benzyl | 0 | - |
| 85 | H | CH₂COOH | NHBenzyl | Benzyl | 0 | - |
| 86 | H | CH₂CONH₂ | NHBenzyl | Benzyl | 0 | - |
| 87 | H | (CH₂)₂0H | NHBenzyl | Benzyl | 0 | - |
| 88 | H | CH(CH₃)OH | NHBenzyl | Benzyl | 0 | - |
| 89 | H | (CH₂)₂COOH | NHBenzyl | Benzyl | 0 | - |
| 90 | H | (CH₂)₂CONH₂ | NHBenzyl | Benzyl | 0 | - |
| 91 | H | CH₂Phenyl | NHBenzyl | Benzyl | 0 | - |
| 92 | H | CH₂-4-OH-Phenyl | NHBenzyl | Benzyl | 0 | - |
| 93 | H | Phenyl | NHBenzyl | Benzyl | 0 | - |
| 94 | H | CH₂-4-Imidazolyl | NHBenzyl | Benzyl | 0 | - |
| 95 | H | CH₂-3-Indolyl | NHBenzyl | Benzyl | 0 | - |
| 96 | H | H | NHBenzyl | C=NH(NH ₂) | 0 | - |
| 97 | H | CH₃ | NHBenzyl | C=NH(NH ₂) | 0 | - |
| 98 | H | CH₂OH | NHBenzyl | C=NH(NH ₂) | 0 | - |
| 99 | H | (CH₂)₂NH₂ | NHBenzyl | C=NH(NH ₂) | 0 | - |
| 100 | H | (CH₂)₃NH₂ | NHBenzyl | C=NH(NH ₂) | 0 | - |
| 101 | H | CH₂NH₂ | NHBenzyl | C=NH(NH ₂) | 0 | - |
| 102 | H | (CH₂)₄NH₂ | NHBenzyl | C=NH(NH ₂) | 0 | - |
| 103 | H | (CH₂)₃NHC=NH(N H₂) | NHBenzyl | C=NH(NH ₂) | 0 | - |
| 104 | H | CH₂CH₃ | NHBenzyl | C=NH(NH ₂) | 0 | - |
| 105 | H | (CH₂)₂CH₃ | NHBenzyl | C=NH(NH ₂) | 0 | - |
| 106 | H | CH(CH₃)₂ | NHBenzyl | C=NH(NH₂) | 0 | - |
| 107 | H | CH₂CH(CH₃)₂ | NHBenzyl | C=NH(NH ₂) | 0 | - |
| 108 | H | CH(CH₃)CH₂CH₃ | NHBenzyl | C=NH(NH ₂) | 0 | - |
| 109 | H | (CH₂)₂SCH₃ | NHBenzyl | C=NH(NH ₂) | 0 | - |
| 110 | H | CH₂COOH | NHBenzyl | C=NH(NH ₂) | 0 | - |
| 111 | H | CH₂CONH₂ | NHBenzyl | C=NH(NH ₂) | 0 | - |
| 112 | H | (CH₂)₂OH | NHBenzyl | C=NH(NH ₂) | 0 | - |
| 113 | H | CH(CH₃)OH | NHBenzyl | C=NH(NH ₂) | 0 | - |
| 114 | H | (CH₂)₂COOH | NHBenzyl | C=NH(NH ₂) | 0 | - |
| 115 | H | (CH₂)₂CONH₂ | NHBenzyl | C=NH(NH ₂) | 0 | - |
| 116 | H | CH₂Phenyl | NHBenzyl | C=NH(NH ₂) | 0 | - |
| 117 | H | CH₂-4-OH-Phenyl | NHBenzyl | C=NH(NH ₂) | 0 | - |
| 118 | H | Phenyl | NHBenzyl | C=NH(NH ₂) | 0 | - |
| 119 | H | CH₂-4-Imidazolyl | NHBenzyl | C=NH(NH ₂) | 0 | - |
| 120 | H | CH₂-3-Indolyl | NHBenzyl | C=NH(NH ₂) | 0 | - |
| 121 | H | H | NHBenzyl | H | 1 | H |
| 122 | H | CH₃ | NHBenzyl | H | 1 | H |
| 123 | H | CH₂OH | NHBenzyl | H | 1 | H |
| 124 | H | (CH₂)₂NH₂ | NHBenzyl | H | 1 | H |
| 125 | H | (CH₂)₃NH₂ | NHBenzyl | H | 1 | H |
| 126 | H | CH₂NH₂ | NHBenzyl | H | 1 | H |
| 127 | H | (CH₂)₄NH₂ | NHBenzyl | H | 1 | H |
| 128 | H | (CH₂)₃NHC=NH(N H₂) | NHBenzyl | H | 1 | H |
| 129 | H | CH₂CH₃ | NHBenzyl | H | 1 | H |
| 130 | H | (CH₂)₂CH₃ | NHBenzyl | H | 1 | H |
| 131 | H | CH(CH₃)₂ | NHBenzyl | H | 1 | H |
| 132 | H | CH₂CH(CH₃)₂ | NHBenzyl | H | 1 | H |
| 133 | H | CH(CH₃)CH₂CH₃ | NHBenzyl | H | 1 | H |
| 134 | H | (CH₂)₂SCH₃ | NHBenzyl | H | 1 | H |
| 135 | H | CH₂COOH | NHBenzyl | H | 1 | H |
| 136 | H | CH₂CONH₂ | NHBenzyl | H | 1 | H |
| 137 | H | (CH₂)₂OH | NHBenzyl | H | 1 | H |
| 138 | H | CH(CH₃)OH | NHBenzyl | H | 1 | H |
| 139 | H | (CH₂)₂COOH | NHBenzyl | H | 1 | H |
| 140 | H | (CH₂)₂CONH₂ | NHBenzyl | H | 1 | H |
| 141 | H | CH₂Phenyl | NHBenzyl | H | 1 | H |
| 142 | H | CH₂-4-OH-Phenyl | NHBenzyl | H | 1 | H |
| 143 | H | Phenyl | NHBenzyl | H | 1 | H |
| 144 | H | CH₂-4-Imidazolyl | NHBenzyl | H | 1 | H |
| 145 | H | CH₂-3-Indolyl | NHBenzyl | H | 1 | H |
| 146 | H | H | NHBenzyl | H | 2 | H |
| 147 | H | CH₃ | NHBenzyl | H | 2 | H |
| 148 | H | CH₂OH | NHBenzyl | H | 2 | H |
| 149 | H | (CH₂)₂NH₂ | NHBenzyl | H | 2 | H |
| 150 | H | (CH₂)₃NH₂ | NHBenzyl | H | 2 | H |
| 151 | H | CH₂NH₂ | NHBenzyl | H | 2 | H |
| 152 | H | (CH₂)₄NH₂ | NHBenzyl | H | 2 | H |
| 153 | H | (CH₂)₃NHC=NH(N H₂) | NHBenzyl | H | 2 | H |
| 154 | H | CH₂CH₃ | NHBenzyl | H | 2 | H |
| 155 | H | (CH₂)₂CH₃ | NHBenzyl | H | 2 | H |
| 156 | H | CH(CH₃)₂ | NHBenzyl | H | 2 | H |
| 157 | H | CH₂CH(CH₃)₂ | NHBenzyl | H | 2 | H |
| 158 | H | CH(CH₃)CH₂CH₃ | NHBenzyl | H | 2 | H |
| 159 | H | (CH₂)₂SCH₃ | NHBenzyl | H | 2 | H |
| 160 | H | CH₂COOH | NHBenzyl | H | 2 | H |
| 161 | H | CH₂CONH₂ | NHBenzyl | H | 2 | H |
| 162 | H | (CH₂)₂OH | NHBenzyl | H | 2 | H |
| 163 | H | CH(CH₃)OH | NHBenzyl | H | 2 | H |
| 164 | H | (CH₂)₂COOH | NHBenzyl | H | 2 | H |
| 165 | H | (CH₂)₂CONH₂ | NHBenzyl | H | 2 | H |
| 166 | H | CH₂Phenyl. | NHBenzyl | H | 2 | H |
| 167 | H | CH₂-4-OH-Phenyl | NHBenzyl | H | 2 | H |
| 168 | H | Phenyl | NHBenzyl | H | 2 | H |
| 169 | H | CH₂-4-Imidazolyl | NHBenzyl | H | 2 | H |
| 170 | H | CH₂-3-Indolyl | NHBenzyl | H | 2 | H |
| 171 | C(CH₃)₃ | H | NHBenzyl | H | 1 | H |
| 172 | C(CH₃)₃ | CH₃ | NHBenzyl | H | 1 | H |
| 173 | C(CH₃)₃ | CH₂OH | NHBenzyl | H | 1 | H |
| 174 | C(CH₃)₃ | (CH₂)₂NH₂ | NHBenzyl | H | 1 | H |
| 175 | C(CH₃)₃ | (CH₂)₃NH₂ | NHBenzyl | H | 1 | H |
| 176 | C(CH₃)₃ | CH₂NH₂ | NHBenzyl | H | 1 | H |
| 177 | C(CH₃)₃ | (CH₂)₄NH₂ | NHBenzyl | H | 1 | H |
| 178 | C(CH₃)₃ | (CH₂)₃NHC=NH(N H₂) | NHBenzyl | H | 1 | H |
| 179 | C(CH₃)₃ | CH₂CH₃ | NHBenzyl | H | 1 | H |
| 180 | C(CH₃)₃ | (CH₂)₂CH₃ | NHBenzyl | H | 1 | H |
| 181 | C(CH₃)₃ | CH(CH₃)₂ | NHBenzyl | H | 1 | H |
| 182 | C(CH₃)₃ | CH₂CH(CH₃)₂ | NHBenzyl | H | 1 | H |
| 183 | C(CH₃)₃ | CH(CH₃)CH₂CH₃ | NHBenzyl | H | 1 | H |
| 184 | C(CH₃)₃ | (CH₂)₂SCH₃ | NHBenzyl | H | 1 | H |
| 185 | C(CH₃)₃ | CH₂COOH | NHBenzyl | H | 1 | H |
| 186 | C(CH₃)₃ | CH₂CONH₂ | NHBenzyl | H | 1 | H |
| 187 | C(CH₃)₃ | (CH₂)₂OH | NHBenzyl | H | 1 | H |
| 188 | C(CH₃)₃ | CH(CH₃)OH | NHBenzyl | H | 1 | H |
| 189 | C(CH₃)₃ | (CH₂)₂COOH | NHBenzyl | H | 1 | H |
| 190 | C(CH₃)₃ | (CH₂)₂CONH₂ | NHBenzyl | H | 1 | H |
| 191 | C(CH₃)₃ | CH₂Phenyl | NHBenzyl | H | 1 | H |
| 192 | C(CH₃)₃ | CH₂-4-OH-Phenyl | NHBenzyl | H | 1 | H |
| 193 | C(CH₃)₃ | Phenyl | NHBenzyl | H | 1 | H |
| 194 | C(CH₃)₃ | CH₂-4-Imidazolyl | NHBenzyl | H | 1 | H |
| 195 | C(CH₃)₃ | CH₂-3-Indolyl | NHBenzyl | H | 1 | H |
| 196 | -CH₂CH₂CH₂- | | NHBenzyl | H | 0 | - |
| 197 | -CH₂CH₂CH₂CH₂- | | NHBenzyl | H | 0 | - |
| 198 | -CH₂CH₂CH₂CH₂CH₂- | | NHBenzyl | H | 0 | - |
| 199 | -CH₂CH₂NHCH₂- | | NHBenzyl | H | 0 | - |
| 200 | -CH₂CH₂OCH₂- | | NHBenzyl | H | 0 | - |
| 201 | -CH₂SCH₂- | | NHBenzyl | H | 0 | - |
| 202 | -CH₂CH₂CH₂- | | NHBenzyl | H | 1 | H |
| 203 | -CH₂CH₂CH₂CH₂- | | NHBenzyl | H | 1 | H |
| 204 | -CH₂CH₂CH₂CH₂CH₂- | | NHBenzyl | H | 1 | H |
| 205 | -CH₂CH₂NHCH₂- | | NHBenzyl | H | 1 | H |
| 206 | -CH₂CH₂OCH₂- | | NHBenzyl | H | 1 | H |
| 207 | -CH₂SCH₂- | | NHBenzyl | H | 1 | H |
| 208 | -CH₂CH₂CH₂- | | NHBenzyl | C=NH(NH 2) | 0 | - |
| 209 | -CH₂CH₂CH₂CH₂- | | NHBenzyl | C=NH(NH ₂) | 0 | - |
| 210 | -CH₂CH₂CH₂CH₂CH₂- | | NHBenzyl | C=NH(NH ₂) | 0 | - |
| 211 | -CH₂CH₂NHCH₂- | | NHBenzyl | C=NH(NH ₂) | 0 | - |
| 212 | -CH₂CH₂OCH₂- | | NHBenzyl | C=NH(NH₂) | 0 | - |
| 213 | -CH₂SCH₂- | | NHBenzyl | C=NH(NH 2) | 0 | - |
| 214 | -CH₂CH₂CH₂- | | NHCH₃ | H | 0 | - |
| 215 | -CH₂CH₂CH₂CH₂- | | NHCH₂CH₃ | H | 0 | - |
| 216 | -CH₂CH_{I}CH_{Z}CH₂CH₂- | | NHC(CH₃)₃ | H | 0 | - |
| 217 | -CH₂CH₂NHCH₂- | | NHCH₃ | H | 0 | - |
| 218 | -CH₂CH₂OCH₂- | | NHCH₂CH₃ | H | 0 | - |
| 219 | -CH₂SCH₂- | | NHC(CH₃)₃ | H | 0 | - |
| 220 | H | H | NHBenzyl | H | 1 | NH₂ |
| 221 | H | CH₃ | NHBenzyl | H | 1 | NH₂ |
| 222 | C(CH₃)₃ | H | NHBenzyl | H | 1 | NH₂ |
| 223 | C(CH₃)₃ | CH₃ | NHBenzyl | H | 1 | NH₂ |
| 224 | H | H | NHBenzyl | C=NH(NH ₂) | 1 | H |
| 225 | C=NH(NH₂) | H | NHBenzyl | H | 1 | H |
| 226 | C=NH(NH₂) | H | NHBenzyl | C=NH(NH ₂) | 1 | H |

**Tabelle 2: Verbindungen der Formel (I), worin R⁶, sofern vorhanden, Wasserstoff bedeutet**

| **Nr** | **R¹** | **R²** | **R³** | **R⁴** | **n** | **X** |
|---|---|---|---|---|---|---|
| 227 | H | H | NHBenzyl | H | 0 | NHCH(C=O)(CH₂)₃NH₂ |
| 228 | H | CH3 | NHBenzyl | H | 0 | NHCH(C=O)(CH₂)₃NH₂ |
| 229 | H | CH₂OH | NHBenzyl | H | 0 | NHCH(C=O)(CH₂)₃NH₂ |
| 230 | H | (CH₂)₂NH₂ | NHBenzyl | H | 0 | NHCH(C=O)(CH₂)₃NH₂ |
| 231 | H | (CH₂)₃NH₂ | NHBenzyl | H | 0 | NHCH(C=O)(CH₂)₃NH₂ |
| 232 | H | CH₂NH₂ | NHBenzyl | H | 0 | NHCH(C=O)(CH₂)₃NH₂ |
| 233 | H | (CH₂)₄NH₂ | NHBenzyl | H | 0 | NHCH(C=O)(CH₂)₃NH₂ |
| 234 | H | (CH₂)₃NHC=NH(N H₂) | NHBenzyl | H | 0 | NHCH(C=0)(CH₂)₃NH₂ |
| 235 | H | CH₂CH₃ | NHBenzyl | H | 0 | NHCH(C=O)(CH₂)₃NH₂ |
| 236 | H | (CH₂)₂CH₃ | NHBenzyl | H | 0 | NHCH(C=O)(CH₂)₃NH₂ |
| 237 | H | CH(CH₃)₂ | NHBenzyl | H | 0 | NHCH(C=O)(CH₂)₃NH₂ |
| 238 | H | CH₂CH(CH₃)₂ | NHBenzyl | H | 0 | NHCH(C=O)(CH₂)₃NH₂ |
| 239 | H | CH(CH₃)CH2-CH3 | NHBenzyl | H | 0 | NHCH(C=O)(CH₂)₃NH₂ |
| 240 | H | (CH₂)₂SCH₃ | NHBenzyl | H | 0 | NHCH(C=O)(CH₂)₃NH₂ |
| 241 | H | CH₂COOH | NHBenzyl | H | 0 | NHCH(C=O)(CH₂)₃NH₂ |
| 242 | H | CH₂CONH₂ | NHBenzyl | H | 0 | NHCH(C=O)(CH₂)₃NH₂ |
| 243 | H | (CH₂)₂OH | NHBenzyl | H | 0 | NHCH(C=O)(CH₂)₃NH₂ |
| 244 | H | CH(CH₃)OH | NHBenzyl | H | 0 | NHCH(C=O)(CH₂)₃NH₂ |
| 245 | H | (CH₂)₂COOH | NHBenzyl | H | 0 | NHCH(C=O)(CH₂)₃NH₂ |
| 246 | H | (CH₂)₂CONH₂ | NHBenzyl | H | 0 | NHCH(C=O)(CH₂)₃NE₂ |
| 247 | H | CH₂Phenyl | NHBenzyl | H | 0 | NHCH(C=O)(CH₂)₃NH₂ |
| 248 | H | CH₂-4-OH-Phenyl | NHBenzyl | H | 0 | NHCH(C=O)(CH₂)₃NH₂ |
| 249 | H | Phenyl | NHBenzyl | H | 0 | NHCH(C=O)(CH₂)₃NH₂ |
| 250 | H | CH₂-4-Imidazolyl | NHBenzyl | H | 0 | NHCH(C=O)(CH₂)₃NH₂ |
| 251 | H | CH₂-3-Indolyl | NHBenzyl | H | 0 | NHCH(C=O)(CH₂)₃NH₂ |
| 252 | H | H | NHBenzyl | C(=NH)NH₂ | 0 | NHCH(C=O)(CH₂)₃NH₂ |
| 253 | H | CH₃ | NHBenzyl | C(=NH)NH₂ | 0 | NHCH(C=O)(CH₂)₃NH₂ |
| 254 | H | H | NHBenzyl | H | 0 | NHCH(C=O)(CH₂)₃NHC (=NH)NH₂ |
| 255 | H | CH₃ | NHBenzyl | H | 0 | NHCH(C=O)(CH₂)₃NHC (=NH)NH₂ |
| 256 | H | H | NHBenzyl | C(=NH)NH₂ | 0 | NHCH(C=O)(CH₂)₃NHC (=NH)NH₂ |
| 257 | H | CH₃ | NHBenzyl | C(=NH)NH₂ | 0 | NHCH(C=O)(CH₂)₃NHC (=NH)NH₂ |
| 258 | H | H | NHBenzyl | H | 1 | NHCH(C=O)(CH₂)₄NH₂ |
| 259 | H | CH₃ | NHBenzyl | H | 1 | NHCH(C=O)(CH₂)₄NH₂ |
| 260 | H | H | NHBenzyl | H | 2 | NHCH(C=O)(CH₂)₅NH₂ |
| 261 | H | CH₃ | NHBenzyl | H | 2 | NHCH(C=O)(CH₂)₅NH₂ |

**Tabelle 3: Verbindungen der Formel (I), worin n 1 bedeutet**

| **Nr** | **R¹** | **R⁶** | **R²** | **R³** | **R⁴** | **X** |
|---|---|---|---|---|---|---|
| 262 | H | NH₂ | H | NHBenzyl | H | NHCH(C=O)(CH₂)₄NH₂ |
| 263 | H | NH₂ | CH₃ | NHBenzyl | H | NHCH(C=O)(CH₂)₄NH₂ |
| 264 | -CH₂CH₂- | | H | NHBenzyl | H | Bindung |
| 265 | -CH₂NH- | | H | NHBenzyl | H | Bindung |
| 266 | -CH₂CH₂CH₂- | | H | NHBenzyl | H | Bindung |
| 267 | -CH₂CH₂NH- | | H | NHBenzyl | H | Bindung |
| 268 | -CH₂CH₂CH₂CH₂- | | H | NHBenzyl | H | Bindung |
| 269 | -CH₂CH₂- | | H | NHBenzyl | C(=NH)NH₂ | Bindung |
| 270 | -CH₂NH- | | H | NHBenzyl | C(=NH)NH₂ | Bindung |
| 271 | -CH₂CH₂CH₂- | | H | NHBenzyl | C(=NH)NH₂ | Bindung |
| 272 | -CH₂CH₂NH- | | H | NHBenzyl | C(=NH)NH₂ | Bindung |
| 273 | -CH₂CH₂CH₂CH₂- | | H | NHBenzyl | C(=NH)NH₂ | Bindung |

Die Verbindungen der Formel (I) können mit Säuren ein- oder mehrwertige, einheitliche oder gemischte Salze bilden, z.B. mit anorganischen Säuren, wie Chlorwasserstoff, Bromwasserstoff, Schwefelsäure oder Phosphorsäure; oder mit geeigneten Carbonsäuren, z.B. aliphatischen Mono- oder Dicarbonsäuren, wie Ameisensäure, Essigsäure, Trifluoressigsäure, Trichloressigsäure, Propionsäure, Glykolsäure, Bernsteinsäure, Fumarsäure, Malonsäure, Maleinsäure, Oxalsäure, Phthalsäure, Zitronensäure, Milchsäure oder Weinsäure; oder mit aromatischen Carbonsäuren, wie Benzoesäure oder Salicylsäure; oder mit aromatisch-aliphatischen Carbonsäuren, wie Mandelsäure oder Zimtsäure; oder mit heteroaromatischen Carbonsäuren, wie Nikotinsäure; oder mit aliphatischen oder aromatischen Sulfonsäuren, wie Methansulfonsäure oder Toluolsulfonsäure. Bevorzugt sind dermatologisch verträgliche Salze. Besonders bevorzugt sind Salze mit Essigsäure und/oder Trifluoressigsäure und/oder Milchsäure.

Die Verbindungen der allgemeine Formel (I) und ihre Salze können als optisch reine Isomere oder als Gemische verschiedener Isomeren, z.B. als Racemate, und/oder als Gemische von Rotameren vorliegen.

Die Verbindungen der allgemeinen Formel I und ihre Salze können erfindungsgemäss dadurch hergestellt werden,, dass man unter Verwendung von an sich in der Peptidchemie bekannten Methoden die Verbindung der Formel (I) vollständig aufbaut, gegebenenfalls die verbleibende(n) Schutzgruppe(n) abspaltet, gegebenenfalls eine freie Aminogruppe alkyliert oder in eine Guanidino Funktion umwandelt und/oder eine freie Carboxylgruppe verestert oder amidiert und/oder eine erhaltene basische Verbindung in ein Säureadditionssalz und /oder ein erhaltenes Säureadditionssalz in die entsprechende konjugate Base oder in ein anderes Salz überführt.

Die erfindungsgemässen Verbindungen der Formel I und deren Salze können nach allgemein üblichen Verfahren zu topisch anwendbaren Zubereitungen verarbeitet werden. Dabei können die Verbindungen der allgemeinen Formel I und ihre Salze im topisch anwendbaren Endprodukt in Konzentrationen verwendet werden, die zwischen 0,5 und 5.000 ppm (w/w), vorzugsweise zwischen 1 und 1000 ppm (w/w), berechnet auf das Gewicht der erfindungsgemässen Verbindung oder eines ihrer Salze und des Trägermaterials bzw. der Trägermaterialien, variieren.

Die erfindungsgemässen Verbindungen der Formel I und ihre Salze können in Form einer Lösung, einer Dispersion, einer Emulsion oder eingekapselt in Träger, wie Makro-, Mikro- oder Nanokapseln, in Liposomen oder Chylomikronen, oder eingeschlossen in Makro-, Mikro- oder Nanoteilchen oder in Mikroschwämme oder absorbiert auf pulverförmigen organischen Polymeren, Talk, Bentonit und anderen mineralischen Trägern verwendet werden.

Die erfindungsgemässen Verbindungen der Formel I und ihre Salze können in jeder für topisch anwendbare Zubereitungen geeigneten Form verwendet werden, wie Emulsionen H/E und E/H, Milch, Lotionen, Salben, gelierende und viskose, spannungsaktive und emulgierende Polymere, Pommaden, Shampoos, Seifen, Gele, Puder, Sticks und Stifte, Sprays, Körperöle, Gesichtsmasken und Pflaster.

Die erfindungsgemässen Verbindungen der Formel I und ihre Salze können zusammen mit jedem anderen üblicherweise verwendeten Inhaltsstoff topisch anwendbarer Zubereitungen verwendet werden, wie Extraktionslipide und/oder Syntheselipide, gelierende und viskose, spannungsaktive und emulgierende Polymere, wasser- oder fettlösliche Wirkprinzipien, Pflanzenextrakte, Gewebeextrakte, Meeresextrakte, Sonnenschutzmittel, Antioxidantien, Feuchthalte- und Barrieremittel sowie Haut revitalisierende Wirkstoffe.

Die erfindungsgemässen Verbindungen können zusammen mit jedem anderen üblicherweise verwendeten topisch anwendbaren Hautpflegewirkstoff verwendet werden. Beispielhaft für zusätzliche Hautpflegewirkstoffe seien Anti-Faltenwirkstoffe/Anti-Atrophiewirkstoffe genannt: Die erfindungsgemässen Zusammensetzung können eine sichere und wirksame Menge von einem oder mehreren Anti-Faltenwirkstoffen oder Anti-Atrophiewirkstoffen enthalten. Beispielhafte Anti-Falten/Anti-Atrophie-Wirkstoffe, die zur Verwendung in den erfindungsgemässen Zusammensetzungen geeignet sind, umfassen schwefelhaltige D- und L-Aminosäuren und ihre Derivate und Salze, insbesondere die N-Acetylderivate, wobei ein bevorzugtes Beispiel hierfür N-Acetyl-L-cystein ist; Thiole, z. B. Ethanthiol; Hydroxysäuren, Phytinsäure, Liponsäure, Lysophosphatidinsäure; Haut-Peelingmittel (z. B. Phenol und dergleichen); Vitamin B3-Verbindungen und Retinoide, welche die Vorteile der erfindungsgemässen Verbindungen der Formel I und ihrer Salze für das Glätten von Falten verbessern.

Nachstehend sollen drei Klassen solcher üblicherweise verwendeten topisch anwendbaren Hautpflegestoffe näher diskutiert werden, nämlich Vitamin B3-Verbindungen, Retinoide und Hydroxysäuren.

### a) Vitamin B3-Verbindungen:

Die erfindungsgemässen Zusammensetzungen können eine sichere und wirksame Menge einer Vitamin B3-Verbindung enthalten. Vitamin B3-Verbindungen sind besonders nützlich zur Regulierung des Hautzustands, wie in WO 97/39733 A1 (veröffentlicht am 30. Oktober 1997) beschrieben. Beispielhafte Derivate der genannten Vitamin B3-Verbindungen schliessen Nicotinsäureester einschliesslich nicht-vasodilatierender Ester von Nicotinsäure (z. B. Tocopherylnicotinat), Nicotinylaminosäuren, Nicotinylalkoholestern von Carbonsäuren, Nicotinsäure-N-oxid und Niacinamid-N-oxid ein.

### b) Retinoide:

Die erfindungsgemässen Zusammensetzungen können auch ein Retinoid enthalten. "Retinoid" schliesst, wie hier verwendet, alle natürlichen und/oder synthetischen Analoga von Vitamin A oder retinolartigen Verbindungen ein, welche die biologische Wirksamkeit von Vitamin A in der Haut besitzen, sowie die geometrischen Isomere und Stereoisomere dieser Verbindungen. Das Retinoid ist vorzugsweise Retinol, ein Retinolester (z. B. C₂- bis C₂₂-Alkylester von Retinol, einschliesslich Retinylpalmitat, Retinylacetat und Retinylpropionat), Retinal und/oder Retinsäure (einschliesslich all-trans-Retinsäure und/oder 13-cis-Retinsäure), insbesondere ein von Retinsäure verschiedenes Retinoid. Andere geeignete Retinoide sind Tocopherylretinoat [Tocopherolester von Retinsäure (trans oder cis)], Adaptalen {6-[3-(1-Adamantyl)-4-methoxyphenyl]-2-naphthoesäure} und Tazaroten (Ethyl-6-[2-(4,4-dimethylthiochroman-6-yl)-ethinyl]nicotinat). Bevorzugte Retinoide sind Retinol, Retinylpalmitat, Retinylacetat, Retinylpropionat, Retinal und Kombinationen davon. Die erfindungsgemässen Zusammensetzungen können eine sichere und wirksame Menge des Retinoids enthalten, so dass die resultierende Zusammensetzung sicher und wirksam zur Regulierung des Zustands von Horngewebe ist, vorzugsweise zur Regulierung von sichtbaren und/oder fühlbaren Diskontinuitäten der Haut, insbesondere zur Regulierung von Zeichen der Hautalterung, ganz besonders zur Regulierung von sichtbaren und/oder fühlbaren Diskontinuitäten der Hautoberflächenbeschaffenheit, die mit Hautalterung zusammenhängen.

### (c) Hydroxysäuren:

Die erfindungsgemässen Zusammensetzungen können eine sichere und wirksame Menge einer Hydroxysäure enthalten. Bevorzugte Hydroxysäuren zur Verwendung in den erfindungsgemässen Zusammensetzungen schliessen α-Hydroxysäuren, wie Milchsäure und Glykolsäure, oder β-Hydroxysäuren wie Salicylsäure und Salicylsäurederivate, z.B. deren Octanoylderivat, ein.

Weiterhin können zusätzliche Peptide, einschliesslich, aber nicht begrenzt auf Di-, Tri-, Tetra-, Penta- und Hexapeptide und Derivate davon den erfindungsgemässen Zusammensetzungen in sicheren und wirksamen Mengen zugefügt werden. "Peptide" bezieht sich hier auf sowohl die natürlich vorkommenden Peptide als auch die synthetisierten Peptide und umfasst auch Peptidomimetika und Metallkomplexe von "Peptiden". Die natürlich vorkommenden und im Handel erhältlichen Zusammensetzungen, die Peptide enthalten, sind hier auch brauchbar.

Zur Verwendung in den erfindungsgemässen Zubereitungen geeignete Dipeptide schliessen Carnosin (β-Ala-His) ein, und geeignete Tripeptide schliessen Gly-His-Lys, Arg-Lys-Arg und His-Gly-Gly ein. Bevorzugte Tripeptide und Derivate davon umfassen Palmitoyl-Gly-His-Lys, das als Biopeptid CL^{™} erworben werden kann. (100 ppm Palmitoyl-Gly-His-Lys, kommerziell erhältlich von Sederma, Frankreich), Peptid CK (Arg-Lys-Arg), Peptid CK+ (Ac-Arg-Lys-Arg-NH₂) und einen Kupferkomplex von Gly-His-Lys oder von His-Gly-Gly, das kommerziell als Lamin von Sigma (St. Louis, Mo., USA) erhältlich ist. Zur Verwendung in den erfindungsgemässen Zubereitungen geeignete Tetrapeptide schliessen Peptid E ein, Arg-Ser-Arg-Lys. Beispiele für Pentapeptide sind Matrixyl (Palmitoyl-Lys-Thr-Thr-Lys-Ser), erhältlich von Sederma, Frankreich, und jene, die in WO 03/037933 beschrieben sind. Ein zur Verwendung in den erfindungsgemässen Zusammensetzungen geeignetes Hexapeptid ist Argireline (Ac-Glu-Glu-Met-Gln-Arg-Arg-NH₂), hergestellt von Lipotec, Spanien.

Die erfindungsgemässen Verbindungen sowie die topisch anwendbaren Zusammensetzungen, die sie enthalten, werden für Hautpflegeprodukte eingesetzt, insbesondere zur Behandlung von mimischen und altersbedingten Falten.

Die nachfolgenden Beispiele sollen die Erfindung erläutern, ohne diese einzuschränken. Benutzte Abkürzungen sind:
- NMR: Magnetische Kernspin Resonanz
- MS: Massenspektrometrie
- Fmoc: 9-Fluorenylmethyloxycarbonyl
- Boc: tert.-Butyloxycarbonyl
- Z: Benzyloxycarbonyl
- Dab: 2,4-Diaminobuttersäure
- Dap: 2,3-Diaminopropionsäure
- Gab: 2-Amino-4-guanidino-buttersäure
- Pro: Prolin [(-)-Pyrrolidin-2-carbonsäure]
- β-Ala: β-Alanin (3-Aminopropionsäure)
- EtOAc: Essigsäureethylester
- DCM: Dichlormethan
- DMF: Dimethylformamid
- TBTU: O-(Benzotrazol-1-yl)-N,N,N',N'-tetramethyluronium-tetrafluorborat
- DIPEA: Diisopropylethylamin
- NMM: N-Methylmorpholin
- HOBt: 1-Hydroxybenzotriazol
- DCC: N,N'-Dicyclohexylcarbodiimid
- TFA: Trifluoressigsäure
- GF/A: Glasfaser-Mikrofilter
- ACN: Acetonitril
- RT: Raumtemperatur
- HPLC: Hochdruck-Flüssigkeits-Chromatographie
- THF: Tetrahydrofuran
- MEM: Minimum essential medium
- M199: Medium 199 (TECOmedical AG)
- Bzl: Benzyl

### Beispiel 1: Herstellung der erfindungsgemässen Verbindungen

Im nachfolgenden Ausführungsbeispiel 1.1 wird die Herstellung eines repräsentativen Vertreters der erfindungsgemässen Verbindungen der Formel (I) und von Salzen solcher Verbindungen beschrieben. Die Analyse der gemäss den Beispielen erhaltenen Eluate und Produkte wurde mit Proton-NMR Spektroskopie, HPLC-Elektrospray-MS oder mittels Elementaranalyse ausgeführt. Die Verbindungen können nach den im Folgenden beschriebenen an sich bekannten Verfahren (allgemeinen Vorschriften von M. Bodanszky "The Practice of Peptide Synthesis" Springer Verlag, 2nd Edition 1994) hergestellt werden. Entsprechend wird die P1-Aminosäure am carboxyterminalen Ende derivatisiert, die α-Aminoschutzgruppe (z.B. eine Boc-Gruppe) wird entfernt. und das Peptid wird mit den in der Peptidsynthese üblichen Reagenzien stufenweise aufgebaut, bis die gewünschte Sequenz vollständig ist. Anschliessend werden die Seitenkettenschutzgruppen (z.B. Z- oder Boc-Funktionen) abgespalten.

### 1.1 Verbindung Nr. 4.5 (vgl. Tabelle 4, unten)

### 1.1.1 Boc-Dab(Z)-NHBzl

1.4g Boc-Dab(Z)-OH wurden in 10ml ACN und 5ml DMF gelöst, 1.35g TBTU und 1.43ml DIPEA wurden zugefügt, anschliessend wurde mit 0.88ml Benzylamin versetzt und 1h bei RT gerührt. Das Lösungsmittel wurde abdestilliert, der Rückstand wurde in EtOAc aufgenommen und die Lösung mit je 3X 10% Na₂CO₃, 10% Zitronensäure und gesättigter NaCl-Lösung gewaschen. Nach Trocknen über Na₂SO₄, Abdestillieren des Lösungsmittels und Trocknen im Vakuumtrockenschrank bei 40°C über Nacht wurden 1.8g öliges Produkt isoliert.

### 1.1.2 H-Dab(Z)-NHBzl x TFA

1.8g der Verbindung 1.1.1 wurden in 15ml DCM gelöst und 5ml TFA zugefügt und die Lösung 30min bei RT gerührt. Nach Abdestillieren des Lösungsmittels wurde das Rohprodukt mittels präparativer HPLC chromatographisch gereinigt und 0.60g der gewünschten Verbindung isoliert. Die theoretische Masse von 342 wurde mit einem Befund von 342 bestätigt.

### 1.1.3 Boc-Pro-Dab(Z)-NHBzl

0.42g Boc-Pro-OH wurden in 8ml THF und 16ml ACN gelöst, 0.63g TBTU und 0.66ml DIPEA zugefügt und bei RT gerührt. Nach 3min wurde eine Lösung bestehend aus 0.60g der Verbindung 1.1.2 und 0.143ml NMM in 10ml THF und 2ml DMF dazugegeben. Nach einstündigem Rühren bei RT wurden die Lösungsmittel abdestilliert und der Rückstand in EtOAc aufgenommen und mit je 3X 10%iger Na₂CO₃-Lösung, 10% Zitronensäure und gesättigter NaCl-Lösung gewaschen. Nach Trocknen über Na₂SO₄, Abdestillieren des Lösungsmittels und Trocknen im Vakuumtrockenschrank bei 40°C über Nacht wurden 0.8g öliges Rohprodukt isoliert.

### 1.1.4 H-Pro-Dab(Z)-NHBzl x HCl

0.8g der obigen Verbindung 1.1.3 wurden in 6ml einer 4M HCl-Lösung in Dioxan gelöst und 1h bei RT gerührt. Nach Abdestillieren des Lösungsmittels wurde das Rohprodukt mittels präparativer HPLC chromatographiert. Dabei erhielt man 0.6g der gewünschten Verbindung. Die theoretische Masse von 439 wurde mit einem Befund von 439 bestätigt.

### 1.1.5 Z-β-Ala-Pro-Dab(Z)-NHBzl

0.34g Z-β-Ala-OH wurden in 10ml THF gelöst und auf 5°C im Eisbad gekühlt, worauf 0.17g HOBt und 0.31g DCC zugefügt wurden. Nach 45min wurde eine Lösung bestehend aus 0.6g der Verbindung 1.1.4 in 10ml THF und 0.14ml NMM zugegeben. Nach Rühren bei RT über 18h wurde das gebildete und ausgefallene Harnstoff-Derivat abfiltiert, das Lösungsmittel abdestilliert und der Rückstand in EtOAc aufgenommen und mit je 3X 10%iger Na₂CO₃-Lösung, 10% Zitronensäure und gesättigter NaCl-Lösung gewaschen. Nach Trocknen über Na₂SO₄ und Abdestillieren des Lösungsmittels wurde das Rohprodukt mit präparativer HPLC chromatographiert. Es wurden 0.5g eines farblosen Oels der gewünschten Verbindung isoliert. Die theoretische Masse von 644 wurde mit einem Befund von 644 bestätigt.

### 1.1.6 H-β-Ala-Pro-Dab-NHBzl x 2TFA

0.5g der Verbindung 1.1.5 wurden in 20ml Essigsäure und 1ml TFA gelöst und die Lösung mit einer Suspension von 100mg 10% Palladiumkohle in 5 ml Wasser versetzt. Wasserstoffgas wurde bis zur Sättigung eingeleitet, und es wurde bis zur vollständigen Umsetzung bei Normaldruck hydriert (ca. 12h). Die Suspension wurde über ein GF/A-Filter filtriert und das Lösungsmittel abdestilliert. Das erhaltene Rohprodukt wurde mittels präparativer HPLC gereinigt und nach Abdestillieren des Lösungsmittels in Wasser gelöst und lyophilisiert.

0.34g der gewünschte Verbindung wurden erhalten. Die theoretische Masse von 376 wurde mit einem Befund von 376 bestätigt.

In analoger Weise können die übrigen Verbindungen der Tabelle 4 hergestellt werden.

**Tabelle 4**

| | | | | | | |
|---|---|---|---|---|---|---|
| 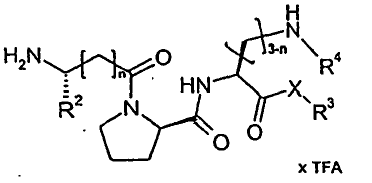 | | | | | | |

| **Nr** | **R²** | **R³** | **R⁴** | **n** | **X** | **MS-Daten** |
|---|---|---|---|---|---|---|
| 4.1 | H | NH-Benzyl | C(=NH)NH₂ | 0 | Bindung | 418 |
| 4.2 | CH₃ | NH-Benzyl | C(=NH)NH₂ | 0 | Bindung | 432 |
| 4.3 | CH₃ | NH-Benzyl | C(=NH)NH₂ | 0 | NHCH(C=O)(CH₂)₃NH -C(=NH)NH₂ | 588 |
| 4.4 | CH₃ | NH-Benzyl | H | 0 | Bindung | 390 |
| 4.5 | H | NH-Benzyl | H | 1 | Bindung | 376 |
| 4.6 | CH₂NH₂ | NH-Benzyl | C(=NH)NH₂ | 0 | Bindung | 447 |
| 4.7 | CH₃ | NH-(CH₂)₂-Phenyl | C(=NH)NH₂ | 0 | Bindung | 446 |
| 4.8 | CH₃ | NH-(4-CH₃O- Benzyl) | C(=NH)NH₂ | 0 | Bindung | 462 |
| 4.9 | H | NHBenzyl | H | 1 | NHCH(C=O)(CH₂)₄NH 2 | |
| 4.10 | H | NHBenzyl | C(=NH)NH₂ | 1 | Bindung | |
| 4.11 | 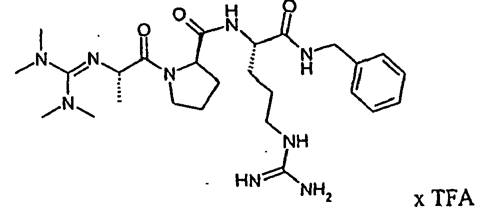 | | | | | 530 |
| 4.12 | 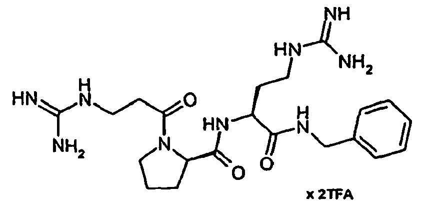 | | | | | |
| 4.13 | 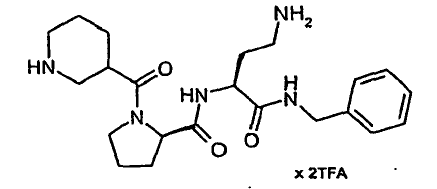 | | | | | |
| 4.14 | 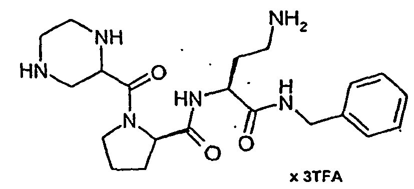 | | | | | |
| 4.15 | 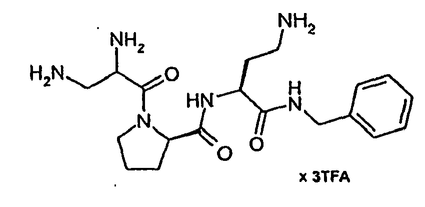 | | | | | |

### Beispiel 2 : Wirksamkeitstest

### 2.1 Material

- normale humane Muskelzellen (Myoblasten) in der 3ten Passage
- Rückenmarkexplantate eines 13Tage alten Rattenembryos mit "dorsalen-root-Ganglionen"
- Kulturmedium: 2/3 MEM und 1/3 M199, 2mM L-Glutamin, Penicillin 50UI/ml, Streptomycin 50µg/ml, Fötales Kälberserum 5%
Kulturbedingungen: 37°C, 5% CO₂

### 2.2 Beschreibung des Tests

Um die Wirksamkeit der erfindungsgemässen Verbindungen zu testen, wurde ein Kokulturmodell von einerseits humanen Muskelzellen und andrerseits Neuronen aus dem Rückenmark von Rattenembryonen etabliert.

Normale humane Muskelzellen (Myoblasten) wurden in mit Gelatine beschichteten 24-well Platten kultiviert, bis sich ein Monolayer von Myofibrillen (aus fusionierten Muskelzellen) gebildet hat. Dann wurde ein Explantat des Rückenmarkes eines 13 Tage alten Rattenembryos mit dorsalen root-Ganglionen auf dem Muskelzell-Monolayer platziert. Nach einem Tag Kokultur sah man die ersten Neuriten aus dem Explant wachsen, um in Kontakt mit den Muskelzellen zu gelangen. Nach 5 Tagen erfolgten die ersten Kontraktionen. Nach drei Wochen in Kokultur hatte sich ein gut ausdifferenziertes Modell von quergestreiften Muskelfasern mit ausgereiften neuromuskulären Verbindungen, vergleichbar mit einer motorischen Endplatte, gebildet (Verbindung von Nerv und Muskel). In diesem Reifestand führten die Myofibrillen regelmässige Kontraktionen durch, und das Modell wurde für die Tests verwendet.

Die Frequenz der Kontraktionen wurde mit einem invertierten Mikroskop mit angeschlossener Videokamera während 30sec. beobachtet, und dann wurden die zu umtersuchenden Verbindungen in den entsprechenden Konzentrationen zugegeben., wobei die Verbindungen in Kulturmedium vorverdünnt wurden. Die Kontraktionen wurden wiederum während 30sec gezählt und zwar 1min und 2Std nach Zugabe der Substanzen. Nach 48Std. Inkubationszeit und einer visuellen Kontrolle der Kokultur wurden die Kontraktionen vor und nach Zugabe von Glucose (Endkonzentration 1g/Liter) erneut gezählt, um auch einen Effekt aufgrund von Glucosemangel auszuschliessen.

### 2.3 Resultate (siehe Tabelle 5)

**Tabelle 5: Prozentuale Veränderung Δ [%] der Anzahl Muskelkontraktionen (gemessen während jeweils 30 Sekunden) 1 Minute, 2 Stunden und 48 Stunden nach Inkubation des Produkts im Vergleich zu der Anzahl Kontraktionen vor der Produktinkubation (Mittelwerte von jeweils 3 Messwerten)**

| **Produkt-Nr** | **Konzentration** | **Δ [%] (1 min)** | **Δ [%] (2 h)** | **Δ [%] (48h)** |
|---|---|---|---|---|
| Kontrolle | - | 92 | 105 | 108 |
| 4.2 | 1.0 mM | 85 | 12 | 60 |
| | 0.5 mM | 93 | 37 | 36 |
| | 0.1 mM | 85 | 78 | 37 |
| 4.3 | 1.0 mM | 25 | 0 | 7 |
| | 0.5 mM | 73 | 1 | 87 |
| | 0.1 mM | 91 | 5 | 88 |
| 4.5 | 1.0 mM | 24 | 0 | 0 |
| | 0.5 mM | 96 | 0 | 0 |
| | 0.1 mM | 86 | 4 | 4 |
| 4.6 | 1.0 mM | 68 | 35 | 0 |
| | 0.5 mM | 78 | 42 | 0 |
| | 0.1 mM | 85 | 68 | 43 |
| 4.7 | 1.0 mM | 105 | 65 | 3 |
| | 0.5 mM | 91 | 0 | 34 |
| | 0.1 mM | 103 | 69 | 74 |
| 4.8 | 1.0 mM | 100 | 58 | 32 |
| | 0.5 mM | 84 | 90 | 78 |
| | 0.1 mM | 85 | 37 | 132 |
| 4.11 | 1.0 mM | 104 | 7 | 0 |
| | 0.5 mM | 105 | 16 | 22 |
| | 0.1 mM | 105 | 75 | 96 |
| Produkt A (Vergleich) | 1.0 mM | 99 | 88 | n.d. |
| | 0.5 mM | 106 | 91 | n.d. |
| | 0.1 mM | 103 | 120 | n.d. |
| | 0.05 mM | 103 | 96 | n.d. |

| | | | | |
|---|---|---|---|---|
| n.d. = nicht ermittelt | | | | |

### Beispiel 3_{:} Formulierung einer Salbe (enthaltend die in der nachstehenden Tabelle 6 angegebenen Inhaltsstoffe)

Prozedur: Die Inhaltstoffe 1-5 (A) werden auf 70°C erhitzt. Die Inhaltstoffe 6-7 (B) werden auf 75°C erhitzt. Unter Rühren wird B zu A gegeben, auf 50°C gekühlt, homogenisiert und auf 30°C abgekühlt. Dann werden die Inhaltstoffe 8-9 (C) und der Inhaltsstoff 10 (D) nacheinander hinzugegeben und kalt gerührt.

**Tabelle 6**

| Nummer | Inhaltsstoff | | % w/w |
|---|---|---|---|
| 1 | (A) | Tego Care 450 | 3.00 |
| 2 | | Cetearylalkohol | 2.25 |
| 3 | | Glycerylstearat | 2.25 |
| 4 | | Cetiol 868 | 10.00 |
| 5 | | Squalane | 5.00 |
| 6 | (B) | Deionisiertes Wasser | 66.99 |
| 7 | | Natriumhyaluronat | 5.00 |
| 8 | (C) | Glycerin | 5.00 |
| 9 | | Phenonip | 0.50 |
| 10 | (D) | Verbindung Nr 4.5 (Tab. 4) | 0.01 |

### Beispiel 4: Formulierung eines Gels (enthaltend die in der nachstehenden Tabelle 7 angegebenen Inhaltsstoffe)

Prozedur: In deionisiertem Wasser (1) werden nacheinander die Inhaltstoffe 2-6 gelöst (A). Mit Inhaltstoff 7 (B) wird auf pH 6.0 gestellt. Dann wird Inhaltstoff 8 (C) hinzugegeben.

**Tabelle 7**

| Nummer | Inhaltsstoff | | % w/w |
|---|---|---|---|
| 1 | (A) | Deionisiertes Wasser | 92.09 |
| 2 | | 1,3-Butandiol | 5.00 |
| 3 | | Phenonip | 0.50 |
| 4 | | Abil B 8843 | 1.50 |
| 5 | | Carboxymethyl Cellulose | 0.15 |
| 6 | | Carbopol Ultrez 10 | 0.75 |
| 7 | (B) | NaOH | |
| 8 | (C) | Verbindung Nr 4.7 (Tab. 4) | 0.01 |

## Patentansprüche

1. Verbindungen der allgemeinen Formel (I) 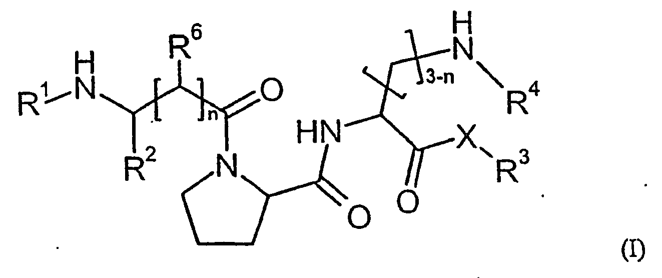 worin
X eine Bindung oder NH-CH(C=O)-(CH₂)₃₊ₙ-NH-R⁵,
n 0, 1 oder 2,
R¹, R⁴ und R⁵ unabhängig voneinander Wasserstoff, gegebenenfalls substituiertes C₁-C₆-Alkyl, Amidino oder Tetra-C₁-C₆-alkylamidinium,
R² Wasserstoff oder gegebenenfalls substituiertes C₁-C₆-Alkyl,
oder
R¹ und R² zusammen mit dem Rest, an den sie gebunden sind, einen 5- bis 7-gliedrigen, gesättigten Ring,
R³ C₁-C₁₂-Alkoxy, C₁-C₁₂-Alkylamino, gegebenenfalls substituiertes Aryl-C₁-C₆-alkylamino, gegebenenfalls substituiertes Heteroaryl-C₁-C₆-alkylamino, gegebenenfalls substituiertes Aryl-C₁-C₆-alkoxy oder gegebenenfalls substituiertes Heteroaryl-C₁-C₆-alkoxy, und
R⁶ Wasserstoff oder, wenn n 1 ist, auch Amino oder zusammen mit R¹ und dem Rest, an den R⁶ und R¹ gebunden sind, einen 5- bis 7-gliedrigen, gesättigten Ring bedeuten.

2. Verbindungen nach Anspruch 1, **dadurch gekennzeichnet, dass** R¹ Wasserstoff bedeutet.

3. Verbindungen nach Anspruch 1 oder 2, **dadurch gekennzeichnet, dass** R² Wasserstoff oder Methyl bedeutet.

4. Verbindungen nach einem der Ansprüche 1-3, **dadurch gekennzeichnet, dass** R³ Aryl-C₁-C₆-alkylamino bedeutet.

5. Verbindungen nach einem der Ansprüche 1-4, **dadurch gekennzeichnet, dass** n 0 oder 1 bedeutet.

6. Verbindungen nach einem der Ansprüche 1-5, **dadurch gekennzeichnet, dass** R¹ Wasserstoff, R² Wasserstoff oder Methyl, R³ Aryl-C₁-C₆-alkylamino und n 0 oder 1 bedeuten.

7. Verbindungen nach einem der Ansprüche 1-6, **dadurch gekennzeichnet, dass** die Verbindungen der Formel (I) als ein- oder mehrwertige, einheitliche oder gemischte Salze mit Säuren vorliegen.

8. Verbindungen nach Anspruch 7, **dadurch gekennzeichnet, dass** sie als Salze mit anorganischen und/oder organischen Säuren vorliegen.

9. Verbindungen nach Anspruch 8, **dadurch gekennzeichnet, dass** sie als Salze mit alphatischen gesättigten und/oder ungesättigten Mono- und/oder Dicarbonsäuren, und/oder mit aromatischen Carbonsäuren, und/oder mit aromatisch-aliphatischen Carbonsäuren, und/oder mit heteroaromatischen Carbonsäuren, und/oder mit aliphatischen und/oder aromatischen Sulfonsäuren vorliegen.

10. Verbindungen nach einem der Ansprüche 7-9, **dadurch gekennzeichnet**, das sie als Salze mit Chlorwasserstoff und/oder Bromwasserstoff und/oder Schwefelsäure und/oder Phosphorsäure und/oder Ameisensäure und/oder Essigsäure und/oder Trifluoressigsäure und/oder Trichloressigsäure und/oder Propionsäure und/oder Glykolsäure und/oder Bernsteinsäure und/oder Fumarsäure und/oder Malonsäure und/oder Maleinsäure und/oder Oxalsäure und/oder Phthalsäure und/oder Zitronensäure und/oder Milchsäure und/oder Weinsäure und/oder Benzoesäure und/oder Salicylsäure und/oder Mandelsäure und/oder Zimtsäure und/oder Nikotinsäure und/oder Methansulfonsäure und/oder Toluolsulfonsäure vorliegen.

11. Verbindungen nach Anspruch 10, **dadurch gekennzeichnet, dass** sie als Salze mit Essigsäure und/oder Trifluoressigsäure und/oder Milchsäure vorliegen.

12. Verbindungen nach einem der Ansprüche 1-11, **dadurch gekennzeichnet, dass** sie als optisch reine Isomere oder als Gemische verschiedener Isomeren und/oder als Gemische von Rotameren vorliegen.

13. H-Ala-Pro-Arg-Arg-NH-benzyl.

14. H-(β-Ala)-Pro-Dab-NH-benzyl.

15. H-Dap-Pro-Arg-NH-benzyl.

16. H-Ala-Pro-Arg-NH-(CH₂)₂-phenyl.

17. H-(β-Ala) -Pro-Gab-NH-benzyl.

18. N-[bis(dimethylamino)methylen]-Ala-Pro-Arg-NH-benzyl.

19. Säureadditionssalze einer oder mehrerer der Verbindungen nach einem der Ansprüche 13-18.

20. Verfahren zur Herstellung der Verbindungen nach einem der Ansprüche 1-19, **dadurch gekennzeichnet, dass** man unter Verwendung von an sich in der Peptidchemie bekannten Methoden die Verbindung der Formel (I) vollständig aufbaut, gegebenenfalls die verbleibende(n) Schutzgruppe(n) abspaltet, gegebenenfalls eine freie Aminogruppe alkyliert oder in eine Guanidino Funktion umwandelt und/oder eine freie Carboxylgruppe verestert oder amidiert und/oder eine erhaltene basische Verbindung in ein Säureadditionssalz und /oder ein erhaltenes Säureadditionssalz in die entsprechende konjugate Base oder in ein anderes Salz überführt.

21. Verwendung von Verbindungen nach einem der Ansprüche 1-19 zur Herstellung von topisch anwendbaren Zusammensetzungen.

22. Verwendung nach Anspruch 21, **dadurch gekennzeichnet, dass** die Verbindungen zur Hautpflege bzw. zur Herstellung von Hautpflegemitteln eingesetzt werden.

23. Verwendung nach Anspruch 22, **dadurch gekennzeichnet, dass** die Verbindungen bzw. die Hautpflegemittel für die Behandlung von mimischen und/oder altersbedingten Falten in der menschlichen Haut eingesetzt werden.

24. Topisch anwendbare Zusammensetzung, **dadurch gekennzeichnet, dass** sie mindestens eine Verbindung nach einem der Ansprüche 1-19, enthält.

25. Zusammensetzung nach Anspruch 24, **dadurch gekennzeichnet, dass** sie mindestens eine Verbindung nach einem der Ansprüche 1-19 in einer Menge im Bereich zwischen 0.5 ppm und 5'000 ppm (w/w), berechnet auf das Gewicht dieser Verbindung(en) und des Trägermaterials bzw. der Trägermaterialien enthält.

26. Zusammensetzung nach Anspruch 25, **dadurch gekennzeichnet, dass** sie mindestens eine Verbindung nach einem der Ansprüche 1-12 in einer Menge im Bereich von zwischen 1 ppm und 1000 ppm (w/w), berechnet auf das Gewicht dieser Verbindung(en) und des Trägermaterials bzw. der Trägermaterialien enthält.

27. Zusammensetzung nach einem der Ansprüche 24-26 als topisch anwendbares Hautpflegemittel.

28. Zusammensetzung nach Anspruch 27 für die Behandlung von mimischen und/oder altersbedingten Falten in der menschlichen Haut.

29. Zusammensetzung nach einem der Ansprüche 24-28, **dadurch gekennzeichnet, dass** diese in topisch anwendbaren Zusammensetzungen üblicherweise verwendete weitere Inhaltsstoffe enthält, welche ausgewählt sind aus der Gruppe umfassend Extraktionslipide und/oder Syntheselipide, gelierende und viskose, spannungsaktive und emulgierende Polymere, wasser- oder fettlösliche Wirkprinzipien, Pflanzenextrakte, Gewebeextrakte, Meeresextrakte, synthetische Produkte, Sonnenschutzmittel, Antioxidantien, Feuchthalte- und Barrieremittel und/oder Haut revitalisierende Wirkstoffe.

30. Topisch anwendbare Zusammensetzung, **dadurch gekennzeichnet, dass** sie
a) mindestens eine Verbindung nach einem der Ansprüche 1-19 und
b) eine sichere und wirksame Menge von mindestens einem zusätzlichen Hautpflegewirkstoff, welcher ausgewählt ist aus der Gruppe der Anti-Faltenwirkstoffe oder Anti-Atrophiewirkstoffe, enthält.

31. Zusammensetzung nach einem der Ansprüche 24-30, **dadurch gekennzeichnet, dass** sie in Form einer Lösung, Dispersion, Emulsion, Milch, Lotion oder Salbe, eines gelierenden und viskosen, spannungsaktiven und emulgierenden Polymeren, einer Pommade, eines Shampoos, einer Seife, eines Gels, Puders, Sticks, Stifts, Sprays oder Körperöls oder eingekapselt in Trägern vorliegt.

32. Zusammensetzung nach Anspruch 31, **dadurch gekennzeichnet, dass** sie in Makro-, Mikro- oder Nanokapseln, in Liposomen oder Chylomikronen, oder eingeschlossen in Makro-, Mikro- oder Nanoteilchen oder in Mikroschwämmen, oder absorbiert auf pulverförmigen organischen Polymeren, Talk, Bentonit und/oder anderen mineralischen Trägern vorliegt.

33. Verfahren zur Behandlung von mimischen und/oder altersbedingten Falten in der menschlichen Haut, **dadurch gekennzeichnet, dass** man eine Verbindung nach einem der Ansprüche 1-19 oder eine Zusammensetzung nach einem der Ansprüche 24-32 auf die Haut appliziert.

34. Verfahren nach Anspruch 33, **dadurch gekennzeichnet, dass** es als kosmetische Behandlung durchgeführt wird.

## Claims

1. Compounds of general formula (I) 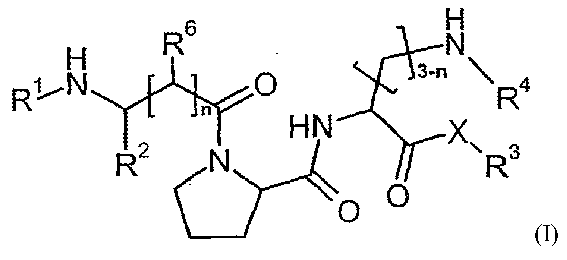 wherein
X is a bond or NH-CH (C=O)-(CH₂)₃₊ₙ-NH-R⁵,
n is 0, 1, or 2,
R¹,R⁴, and R⁵ independently stand for hydrogen, optionally substituted C₁-C₆ alkyl, amidino, or tetra-C₁-C₆-alkylamidinium,
R² is hydrogen or optionally substituted C₁-C₆ alkyl,
or
R¹ and R² together with the radical to which they are bonded stand for a 5- to 7-membered saturated ring,
R³ is C₁-C₁₂-alkoxy, C₁-C₁₂-alkylamino, optionally substituted aryl-C₁-C₆-alkylamino, optionally substituted heteroaryl-C₁-C₆-alkylamino, optionally substituted aryl-C₁-C₆-alkoxy, or optionally substituted heteroaryl-C₁-C₆-alkoxy, and
R⁶ is hydrogen or, if n is 1, is also amino, or together with R¹ and the radical to which R⁶ and R¹ are bonded stand for a 5- to 7-membered saturated ring.

2. Compounds according to Claim 1, **characterized in that** R¹ stands for hydrogen.

3. Compounds according to Claim 1 or 2, **characterized in that** R² stands for hydrogen or methyl.

4. Compounds according to one of Claims 1 through 3, **characterized in that** R³ stands for aryl-C₁-C₆-alkylamino.

5. Compounds according to one of Claims 1 through 4, **characterized in that** n stands for 0 or 1.

6. Compounds according to one of Claims 1 through 5, **characterized in that** R¹ stands for hydrogen, R² stands for hydrogen or methyl, R³ stands for aryl-C₁-C₆-alkylamino, and n stands for 0 or 1.

7. Compounds according to one of Claims 1 through 6, **characterized in that** the compounds of formula (I) are present as monovalent or polyvalent, single or mixed salts with acids.

8. Compounds according to Claim 7, **characterized in that** said compounds are present as salts with inorganic and/or organic acids.

9. Compounds according to Claim 8, **characterized in that** said compounds are present as salts with aliphatic saturated and/or unsaturated mono- and/or dicarboxylic acids, and/or with aromatic carboxylic acids, and/or with aromatic-aliphatic carboxylic acids, and/or with heteroaromatic carboxylic acids, and/or with aliphatic and/or aromatic sulfonic acids.

10. Compounds according to one of Claims 7 through 9, **characterized in that** said compounds are present as salts with hydrogen chloride and/or hydrogen bromide and/or sulfuric acid and/or phosphoric acid and/or formic acid and/or acetic acid and/or trifluoroacetic acid and/or trichloroacetic acid and/or propionic acid and/or glycolic acid and/or succinic acid and/or fumaric acid and/or malonic acid and/or maleic acid and/or oxalic acid and/or phthalic acid and/or citric acid and/or lactic acid and/or tartaric acid and/or benzoic acid and/or salicylic acid and/or mandelic acid and/or cinnamic acid and/or nicotinic acid and/or methanesulfonic acid and/or toluenesulfonic acid.

11. Compounds according to Claim 10, **characterized in that** said compounds are present as salts with acetic acid and/or trifluoroacetic acid and/or lactic acid.

12. Compounds according to one of Claims 1 through 11, **characterized in that** said compounds are present as optically pure isomers or as mixtures of various isomers, and/or as mixtures of rotamers.

13. H-Ala-Pro-Arg-Arg-NH-benzyl.

14. H-(β-Ala)-Pro-Dab-NH-benzyl.

15. H-Dap-Pro-Arg-NH-benzyl.

16. H-Ala-Pro-Arg-NH-(CH₂)₂-phenyl.

17. H-(β-Ala)-Pro-Gab-NH-benzyl.

18. N-[bis(dimethylamino)methylene]-Ala-Pro-Arg-NH-benzyl.

19. Acid addition salts of one or more of the compounds according to one of Claims 13 through 18.

20. Method for preparing the compounds according to one of Claims 1 through 19, **characterized in that** the compound of formula (I) is completely decomposed, using methods known as such in peptide chemistry, the remaining protective group(s) is/are optionally cleaved, a free amino group is optionally alkylated or converted to a guanidino function, and/or a free carboxyl group is esterified or amidated, and/or an obtained basic compound is converted to an acid addition salt and/or an obtained acid addition salt is converted to the corresponding conjugate base or to another salt.

21. Use of compounds according to one of Claims 1 through 19 for preparing topically applicable compositions.

22. Use according to Claim 21, **characterized in that** the compounds are used for skin care or for producing skin care agents.

23. Use according to Claim 22, **characterized in that** the compounds or the skin care agents are used for the treatment of mimic and/or age-related wrinkles in the human skin.

24. Topically applicable composition, **characterized in that** said composition contains at least one compound according to one of Claims 1 through 19.

25. Composition according to Claim 24, **characterized in that** said composition contains at least one compound according to one of Claims 1 through 19 in a quantity ranging between 0.5 and 5000 ppm (w/w), calculated based on the weight of this/these compound(s) and of the carrier material(s).

26. Composition according to Claim 25, **characterized in that** said composition contains at least one compound according to one of Claims 1 through 12 in a quantity ranging between 1 ppm and 1000 ppm (w/w), calculated based on the weight of this/these compound(s) and of the carrier material(s).

27. Composition according to one of Claims 24 through 26 as a topically applicable skin care agent.

28. Composition according to Claim 27 for the treatment of mimic and/or age-related wrinkles in the human skin.

29. Composition according to one of Claims 24 through 28, **characterized in that** said composition contains additional ingredients commonly used in topically applicable preparations, which are selected from the group comprising extraction lipids and/or synthesis lipids, gelling and viscous, surface-active and emulsifying polymers, water- or fat-soluble active principles, plant extracts, tissue extracts, marine extracts, synthetic products, sunscreens, antioxidants, moisturizers and barrier agents, and/or skin-revitalizing active substances.

30. Topically applicable composition, **characterized in that** said composition contains
a) at least one compound according to one of Claims 1 through 19 and
b) a safe and effective quantity of at least one additional skin care active substance selected from the group of anti-wrinkle active substances or anti-atrophy active substances.

31. Composition according to one of Claims 24 through 30, **characterized in that** said composition is present in the form of a solution, dispersion, emulsion, milk, lotion, or ointment, a gelling and viscous, surface-active and emulsifying polymer, a pomade, shampoo, soap, gel, powder, stick, pencil, spray, or body oil, or incorporated into carriers.

32. Composition according to Claim 31, **characterized in that** said composition is present in macro-, micro-, or nanocapsules, in liposomes or chylomicrons, or incorporated into macro-, micro-, or nanoparticles or in microsponges, or absorbed onto powdered organic polymers, talc, bentonite, and/or other mineral carriers.

33. Method for the treatment of mimic and/or age-related wrinkles in the human skin, **characterized in that** a compound according to one of Claims 1 through 19 or a composition according to one of Claims 24 through 32 is applied to the skin.

34. Method according to Claim 33, **characterized in that** said method is carried out as a cosmetic treatment.

## Revendications

1. Composés de la formule générale (I) : 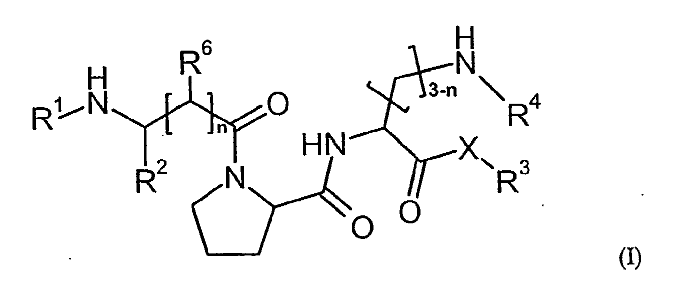 dans laquelle :
- X représente une liaison ou NH-CH(C=O)-(CH2)₃₊ₙ-NH-R⁵;
- n vaut 0, 1 ou 2 ;
- R¹, R⁴ et R⁵ représentent indépendamment l'un de l'autre hydrogène, alkyle en C₁-C₆ éventuellement substitué, amidino ou tétra-(alkyl en C₁-C₆)-amidinium ;
- R² représente hydrogène ou alkyle en C₁-C₆ éventuellement substitué ;
où
- R¹ et R² forment, conjointement avec le reste sur lequel ils sont liés, un noyau saturé à 5 à 7 chaînons ;
- R³ représente alcoxy en C₁-C₁₂, alkylamino en C₁-C₁₂, aryl-(alkyl en C₁-C₆) amino éventuellement substitué, hétéroaryl-(alkyl en C₁-C₆) amino éventuellement substitué, aryl-(alcoxy en C₁-C₆) éventuellement substitué ou hétéroaryl-(alcoxy en C₁-C₆) éventuellement substitué ; et
- R⁶ représente hydrogène ou, lorsque n vaut 1, également amino ou conjointement avec R¹ et le reste sur lequel R⁶ et R¹ sont liés, un noyau saturé à 5 à 7 chaînons.

2. Composés selon la revendication 1, **caractérisés par le fait que** R¹ représente hydrogène.

3. Composés selon l'une des revendications 1 ou 2, **caractérisés par le fait que** R² représente hydrogène ou méthyle.

4. Composés selon l'une des revendications 1 à 3, **caractérisés par le fait que** R³ représente aryl-(alkyl en C₁-C₆) amino.

5. Composés selon l'une des revendications 1 à 4, **caractérisés par le fait que** n vaut 0 ou 1.

6. Composés selon l'une des revendications 1 à 5, **caractérisés par le fait que** R¹ représente hydrogène, R² représente hydrogène ou méthyle, R³ représente aryl-(alkyl en C₁-C₆)-amino et n vaut 0 ou 1.

7. Composés selon l'une des revendications 1 à 6, **caractérisés par le fait que** les composés de formule (I) sont présents sous la forme de sels monovalents ou multivalents, simples ou mixtes, avec les acides.

8. Composés selon la revendication 7, **caractérisés par le fait qu'**ils se présentent sous la forme de sels avec les acides inorganiques et/ou organiques.

9. Composés selon la revendication 8, **caractérisés par le fait qu'**ils se présentent sous la forme de sels avec les acides mono- et/ou dicarboxyliques saturés et/ou insaturés aliphatiques, et/ou avec les acides carboxyliques aromatiques, et/ou avec les acides carboxyliques aromatiques-aliphatiques, et/ou avec les acides carboxyliques hétéroaromatiques, et/ou avec les acides sulfoniques aliphatiques et/ou aromatiques.

10. Composés selon l'une des revendications 7 à 9, **caractérisés par le fait qu'**ils se présentent sous la forme de sels avec l'acide chlorhydrique et/ou l'acide bromhydrique et/ou l'acide sulfurique et/ou l'acide phosphorique et/ou l'acide formique et/ou l'acide acétique et/ou l'acide trifluoroacétique et/ou l'acide trichloroacétique et/ou l'acide propionique et/ou l'acide glycolique et/ou l'acide succinique et/ou l'acide fumarique et/ou l'acide malonique et/ou l'acide maléique et/ou l'acide oxalique et/ou l'acide phtalique et/ou l'acide citrique et/ou l'acide lactique et/ou l'acide tartrique et/ou l'acide benzoïque et/ou l'acide salicylique et/ou l'acide mandélique et/ou l'acide cinnamique et/ou l'acide nicotinique et/ou l'acide méthane sulfonique et/ou l'acide toluène sulfonique.

11. Composés selon la revendication 10, **caractérisés par le fait qu'**ils se présentent sous la forme de sels avec l'acide acétique et/ou l'acide trifluoroacétique et/ou l'acide lactique.

12. Composés selon l'une des revendications 1 à 11, **caractérisés par le fait qu'**ils se présentent sous la forme d'isomères optiquement purs ou sous la forme de mélanges avec de différents isomères et/ou sous la forme de mélanges avec de rotamères.

13. H-Ala-Pro-Arg-Arg-NH-benzyle.

14. H-(β-Ala)-Pro-Dab-NH-benzyle.

15. H-Dap-Pro-Arg-NH-benzyle.

16. H-Ala-Pro-Arg-NH-(CH₂)₂-phényle.

17. H-(β-Ala)-Pro-Gab-NH-benzyle.

18. N-[bis(diméthylamino)méthylène]-Ala-Pro-Arg-NH-benzyle.

19. Sels d'addition avec les acides d'un ou plusieurs des composés tels que définis à l'une des revendications 13 à 18.

20. Procédé de fabrication des composés tel que définis à l'une des revendications 1 à 19, **caractérisé par le fait que** l'on forme entièrement le composé de formule (I) avec utilisation des méthodes connues en soi dans la chimie des peptides, le cas échéant que l'on dissocie le ou les groupes protecteurs restants, le cas échéant que l'on alkyle ou l'on transforme en une fonction guanidino un groupe amino libre et/ou que l'on réalise l'estérification ou l'amidification d'un groupe carboxyle libre et/ou que l'on transforme un composé basique obtenu en un sel d'addition avec un acide et/ou un sel d'addition avec un acide obtenu en la base conjuguée correspondante ou en un autre sel.

21. Utilisation de composés tels que définis à l'une des revendications 1 à 19 pour la fabrication de compositions applicables par voie topique.

22. Utilisation selon la revendication 21, **caractérisée par le fait que** les composés sont utilisés pour les soins dermatologiques ou pour la préparation d'agents de soins dermatologiques.

23. Utilisation selon la revendication 22, **caractérisée par le fait que** les composés ou les agents de soins dermatologiques sont utilisés pour le traitement de rides d'expression et/ou de celles liées au vieillissement de la peau humaine.

24. Composition applicable par voie topique, **caractérisée par le fait qu'**elle contient au moins un composé tel que défini à l'une des revendications 1 à 19.

25. Composition selon la revendication 24, **caractérisée par le fait qu'**elle contient au moins un composé tel que défini à l'une des revendications 1 à 19 dans une quantité se situant dans la plage entre 0,5 et 5 000 ppm (p/p), calculée sur la base du poids de ce ou de ces composés et de la matière de support ou des matières de support.

26. Composition selon la revendication 25, **caractérisée par le fait qu'**elle contient au moins un composé tel que défini à l'une des revendications 1 à 12 dans une quantité se situant dans la plage d'entre 1 ppm et 1 000 ppm (p/p) calculée sur la base du poids de ce ou de ces composés et de la matière de support ou des matières de support.

27. Composition selon l'une des revendications 24 à 26 comme agent de soins de la peau applicable par voie topique.

28. Composition selon la revendication 27 pour le traitement de rides d'expression et/ou de celles liées au vieillissement de la peau humaine.

29. Composition selon l'une des revendications 24 à 28, **caractérisée par le fait que** celle-ci contient d'autres ingrédients utilisés de manière habituelle dans des compositions applicables par voie topique, lesquels sont choisis dans le groupe constitué par les lipides d'extraction et/ou les lipides de synthèse, les polymères gélifiants et visqueux, tenseurs et émulsifiants, les principes actifs solubles dans l'eau ou dans l'huile, les extraits végétaux, les extraits tissulaires, les extraits marins, les produits de synthèse, les agents de protection solaire, les anti-oxydants, les agents humectants et de barrière et/ou les agents de revitalisation de la peau.

30. Composition applicable par voie topique, **caractérisée par le fait qu'**elle contient :
a) au moins un composé tel que défini à l'une des revendications 1 à 19 ; et
b) une quantité sans danger et efficace d'au moins un agent actif de soins dermatologiques supplémentaire, lequel est choisi dans le groupe constitué par les agents anti-rides ou les agents anti-atrophie.

31. Composition selon l'une des revendications 24 à 30, **caractérisée par le fait qu'**elle se présente sous la forme d'une solution, d'une dispersion, d'une émulsion, d'un lait, d'une lotion ou d'une crème, d'un polymère gélifiant et visqueux, tenseur et émulsifiant, une pommade, un shampooing, un savon, un gel, une poudre, un bâton, un crayon, une pulvérisation ou une huile corporelle ou encapsulée dans des supports.

32. Composition selon la revendication 31, **caractérisée par le fait qu'**elle se présente dans des macro-, micro- ou nano-capsules, dans des liposomes ou des chylomicrons, ou enfermée dans des macro-, micro- ou nano-particules ou dans des microéponges, ou absorbée sur des polymères organiques pulvérulents, du talc, de la bentonite et/ou d'autres supports minéraux.

33. Procédé de traitement de rides d'expression et/ou de celles liées au vieillissement de la peau humaine, **caractérisé par le fait que** l'on applique sur la peau un composé tel que défini à l'une des revendications 1 à 19 ou une composition telle que définie à l'une des revendications 24 à 32.

34. Procédé selon la revendication 33, **caractérisé par le fait qu'**il est réalisé sous la forme d'un traitement cosmétique.
